# EUROPEAN PATENT SPECIFICATION

(11) **EP 0 402 431 B1**
(45) Date of publication and mention of the grant of the patent: **07.12.1994**
(21) Application number: 89910905.2
(22) Date of filing: 26.09.1989
(51) Int. Cl.: C12M 1/14, G01N 33/483

(54) **TESTING BACTERIOLOGICAL ACTIVITY**
TESTEN BAKTERIOLOGISCHER AKTIVITÄT
TEST DE L'ACTIVITE BACTERIOLOGIQUE

(30) Priority: 27.09.1988 GB 8822586
(43) Date of publication of application: 19.12.1990
(73) Proprietor: Don Whitley Scientific Limited, Shipley West Yorkshire, BD17 7SE (GB)
(72) Inventor: WHITLEY, Donald, Charles, West Yorkshire BD22 0QT (GB)
(74) Representative: Maguire, Peter Albert
(86) International application number: GB8901131
(87) International publication number: WO9003424

(56) References cited:
- EP-A- 233 024
- GB-A- 1 433 887
- US-A- 3 890 201
- US-A- 3 984 766
- US-A- 4 160 205
- JOURNAL OF CLINICAL PATHOLOGY, vol. 36, no. 11, 1983; O'GRADY et al., pp. 1329-1332#
- JOURNAL OF CLINICAL MICROBIOLOGY, vol. 5, no. 1, 1977; pp. 51-57#
- JOURNAL OF CLINICAL MICROBIOLOGY, vol. 1, no. 1, 1975; pp. 25-29#

## Description

This invention relates to systems for testing bacteriological activity.

It is well established that bacteriological activity of samples added to suitable liquid media, usually of or including nutrient, can be monitored and measured by changes of electrical impedance of the media. Basic requirements of systems and apparatus for bacteriological impedance measurement include provisions of suitable test cells and related electrodes within cell contents; of suitable host environment for the test cells including temperature control and which may be different for different samples; of reliable electronics for actual impedance measurement; of sample and test cell handling before during and after bacteriological activity operations; and of handling and presentation of data concerning such operations and results thereof.

Such systems for testing bacteriological activity are disclosed in US-A-4 160 205, EP-A-0 233 024 and GB-A-1 433 887. A system which uses opacity monitoring of the growth of bacteria is described in J. Clin. Pathol. 36, 1229-1232 (1983).

It is believed that previously available systems and apparatus can be improved upon significantly and objects of this invention are to make advantageous provisions in various respects and represented by various aspects hereof.

One aspect, concerning the host environment for test cells, provides that plural test cell accommodations are disposed spaced, typically in rows and columns, within heat conductive solid state material, such as a heat-conducting block (for which metal, conveniently aluminium, is suitable), and related heating provision, conveniently accommodation for electrical heater device(s) also within said material, so that accommodated test cells are surrounded by said material maintained at a suitable temperature.

Preferred solid host blocks are apertured to provide said accommodations, typically as tubular through-holes for tubular test cells. Such tubular test cells can advantageously extend between parts of releasable electrical connections fixed to one of opposite faces of the block and closures of the test cells at or protruding from the other of the opposite faces of the block. Electrical connections having releasable and engagable parts, say of plug-and-socket type, and having one part fixed to the block and the other to the end of a tubular test cell and carrying internal electrode(s) thereof, are satisfactory and particularly readily used.

Host blocks hereof are readily insulated against heat loss, preferably by material of a test unit housing which may also house electronics related to the test cells and temperature control of the block and to production of data signals. At least some of such electronics may be in another compartment of the housing heat-shielded from the block, but some may be deliberately located so as to follow the temperature of the block.

One major advantage of solid host blocks, as compared with prior liquid temperature control baths or gas (usually air) incubators, is the possibility for presentation of test cell receiver apertures let into a block surface disposed at an angle convenient to the user, further preferably in a generally matching housing also presenting other control/display facilities to the user at a surface with the same or a similar angle.

Another aspect concerns test cells, particularly aperture closures, typically for otherwise free or open ends thereof, and provides an overfitting dished cap having a slip-on fit and an aperture-edge-engaging seal, the cap being displaceable by build up of gas pressure within the test cell so as to relieve that pressure, which may result from bacteriological activity. Preferably, such cap further has internal spacers to assure both accurate location of the seal, which may conveniently be domed, and availability of symmetrical gas relief passages about the cap dishing, even for a tubular test cell that is inclined during operation.

Prior test cells have had electrodes that are either moulded-in, particularly for non-reusable cells and/or formed of noble metal, usually as platings on caps of blind cells. We, however, find that electrodes of stainless steel are both reusable and economic, as well as being satisfactory in operation, particularly as they are inherently hard-wearing and readily cleaned/sterilised for re-use. Moreover, incorporation into a plug or cap for the bottom of a test cell particularly aids removal, cleaning (including sterilisation) and re-use, and such plug may be or be part of an electrical fitting part.

Further aspects of this invention concern modularity of units each having a thermal block affording a bank of test cell accommodations and related control provisions local thereto; extensibility of a system to employ a plurality of such units, particularly in conjunction with a computer-type central console and an extensible data/control polling communication provision; uniform and readily understood visual presentation of on-going operations using formatted displays, preferably also for corresponding results; simplicity of electronics for each modular unit, particularly through avoidance of undue reliance on solid-state semiconductor control means that is temperature sensitive and/or providing for at least some of such means to follow temperature changes; and general handling of test cells in an orderly manner contributing to efficiency of overall system deployment and operation.

Practical implementation of one embodiment of this invention will now be described, by way of example, with reference to the accompanying drawings, in which:
Figure 1 is a perspective view of a host block;
Figure 2A and 2B are perspective views of upper and lower casing parts;
Figure 3 is a perspective view of a lid for the casing of Figure 2;
Figure 4 is a fragmental section through the host block and a test cell;
Figure 5 is a section through a cap of a test cell;
Figures 6A and B are bottom and part sectional views (on line B-B) of a seal for the cap;
Figures 7A, B, C are top, sectional and bottom views of the cap without its seal;
Figure 8 is a block diagram of one module or apparatus hereof;
Figure 9 is an overall block diagram of a multi-module bacteriological activity monitoring system; and
Figure 10 is a diagram indicating preferred display formatting.

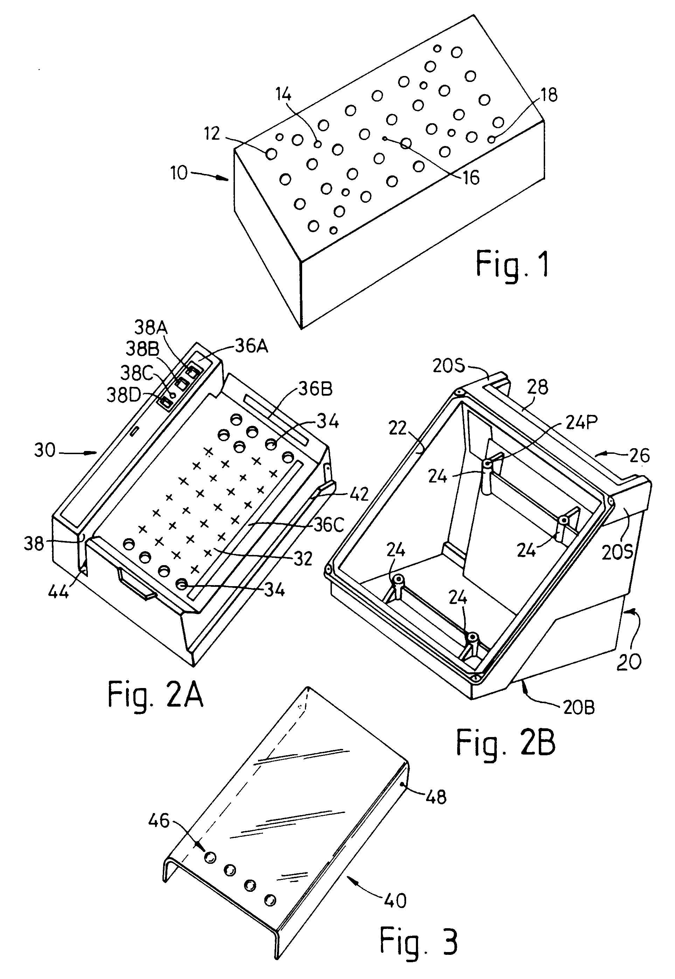

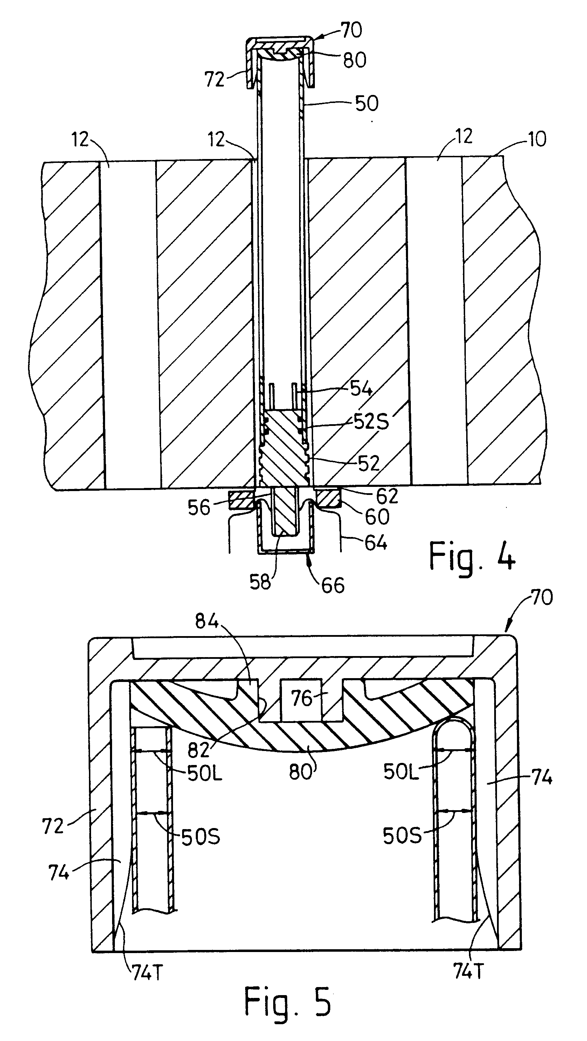

In the drawings, referring first to Figures 1 to 5, a host block 10 is of generally cuboid form pierced by through-holes 12, shown in a regular array of thirty two (actually in a block 200mm x 400mm and 100 mm thick), for accommodating test cells 50 (see Figure 4), shown tubular, with small clearance, (typically about 0.5mm in holes 12 of 16.5mm diameter). The host block 10 is heat conducting material and forms a thermal store. It is conveniently heated by elongate electrical heater elements housed in further holes 14, shown as four in number and also regularly disposed in the body of the block 10, and conveniently counterbored (typically to a diameter of 20mm from a pilot hole of 4.5mm diameter). Another hole 16, shown central, serves for housing temperature sensing means, conveniently a thermistor, and is usually blind (typically of about 3.2mm diameter and 50mm deep). Counterboring (typically to 20mm) from the bottom of the block 10 serves for taking suitable mounting holders, say of plug-in type, for the heaters/thermistor. Further through-holes 18, shown as four in number and conveniently of 9mm diameter, if desired counterbored (say to 14mm), are provided for mounting the block 10.

The block 10 will maintain a remarkably even temperature throughout as required for consistency multiple sample testing, if it is surrounded by thermally insulating material. It is particularly preferred herein that such thermal insulation be afforded by a housing or casing made from suitable material, for example 10 mil high-pressure expanded polyurethane foam, and which is readily moulded to an appropriate shape and attractive finish, for example of suitable texturing effect.

Figures 2A and 2B show such a casing in lower and upper parts 20,30. The lower part 20 has a sloping open front 22 to be covered by the upper part 30 which is of generally dished, inverted tray-like form overfitting onto the open front 22 of the lower part 20. The lower part 20 further has pierced mounting studs 24 to take screws or bolts by which the host block 10 is mounted with its mounting holes 18 registering such piercings 24P of the studs 24.

As so mounted, the block 10 will also be canted to match the sloping entry to the lower casing part 20, the latter being squared off to an overall triangular sectional shape to stand on its base 20B, and have an inset recess 26 behind its back between rear extensions of its sides 20S. Also, a vertical circuit board mounting panel is shown at 28 so that some electronics of the board (not shown) will be within the casing 20,30 and thus follow the temperature of the block 10, but other electrical etc components can be effectively external in the recess 26.

The upper casing part 30 has a panel 32 pierced at 34 in registration with the cell-accommodating holes 12 of a block 10 when mounted in the lower casing part 20. Recesses 36A,B,C serve for neatly affixing a membrane panel and labels showing coordinates for the test cells, respectively. The recess 36A is shown in an upstand 38 that is pierced at 38A,8,C,D for various controls/indicators required or useful in operation and associated with electrical/electronic provisions.

In use, the test cells 50 will project from the block 10, in fact pass through the holes 34 in the upper casing parts 30, and a see-through cover 40 is provided, conveniently of perspex, and shown as of simple channel shape to over-fit the panel 32 at one side onto the rebate 42 and at the other side into slot 44, Provision is shown at 46 for suitable markings or graphics covering each of the test cells at corresponding positions, Preferably, the lid 40 is hinged to the upper covering part 30 at 48.

Reverting to the host block 10 and the test cells 50, Figure 3 shows one through-hole 12 occupied by a tubular test cell 50. At its lower end, the test cell 50 has a plug 52 entering and sealing to the inner surface of the cell 50 by 'O' ring seals 52S. The plug 52 has electrodes 54, which can be of stainless steel, protruding from one end into the interior of the cell tube 50 and contacts 56 protruding from its other end exterior of the cell tube 50, actually on sides of a supporting extension 58. At each test cell hole 12, and at its bottom side, the block 10 has an electrical socket 60 secured thereto at 62 with contacts 64 supported at 66 and making electrical connection with the contacts 56 on the extension 58 of the plug 52 in the bottom of the test cell 50. Moreover, mutual engagement of the plug 52 in the socket 60 affords mechanical support for the test cell 50, particularly facilitating insertion and removal of the cell 50 from the front of the upper casing part 30 with the lid 40 raised for access. The plug 52 is readily demounted from the tube 50 for cleaning purposes.

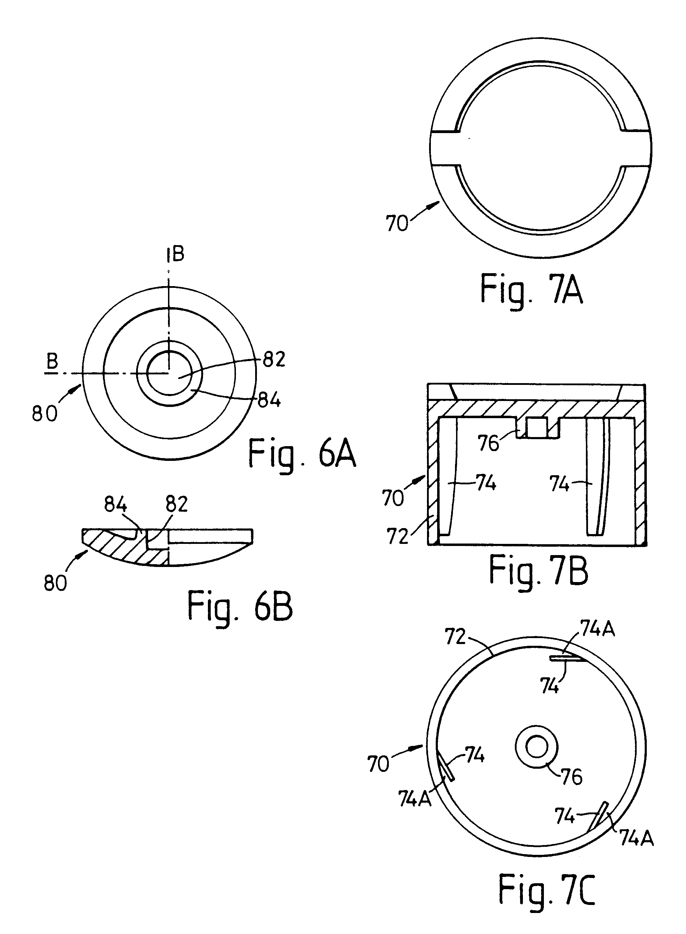

At its other end, which will protrude from the upper casing part 30 in use, each test cell (50) has a cap 70 that is of dished form presenting a skirt 72 fitting about the otherwise free end of the test cell 50. It is particularly advantageous in order to cope with variations of test cell tube diameter, see 50L and 50S in Figure 5, for the cap 70 to have evenly spaced fins 74 extending from its interior at acute angles 74A (see Figure 7C) and with entry tapers 74T. Such arrangement of fins 74 also, of course, assures that there is an even spacing between the cap skirt 70 and the exterior of the test cell 50, which is advantageous for release of gaseous products of bacteriological activity within the cells 50 when an end seal thereto is broken.

Such end seal is provided by a domed pad 80, preferably of soft resilient material, for example silicone rubber, and shown fitting inside the fins 74, see Figures 5 and 6. Interference fitting of a central recess 82 inside an annular rib 84 onto a central annular boss interior 76 of the cap 70, is preferred, preferably with substantial retention grip. Cell end sealing by the pad 80 is readily disturbed by the build up of pressure inside the test cell 50, so as to enable gas escape, and may be equally readily restored, even automatically where the weight of the cap 50 overcomes any light gripping of the fins 74 on the test cells 50.

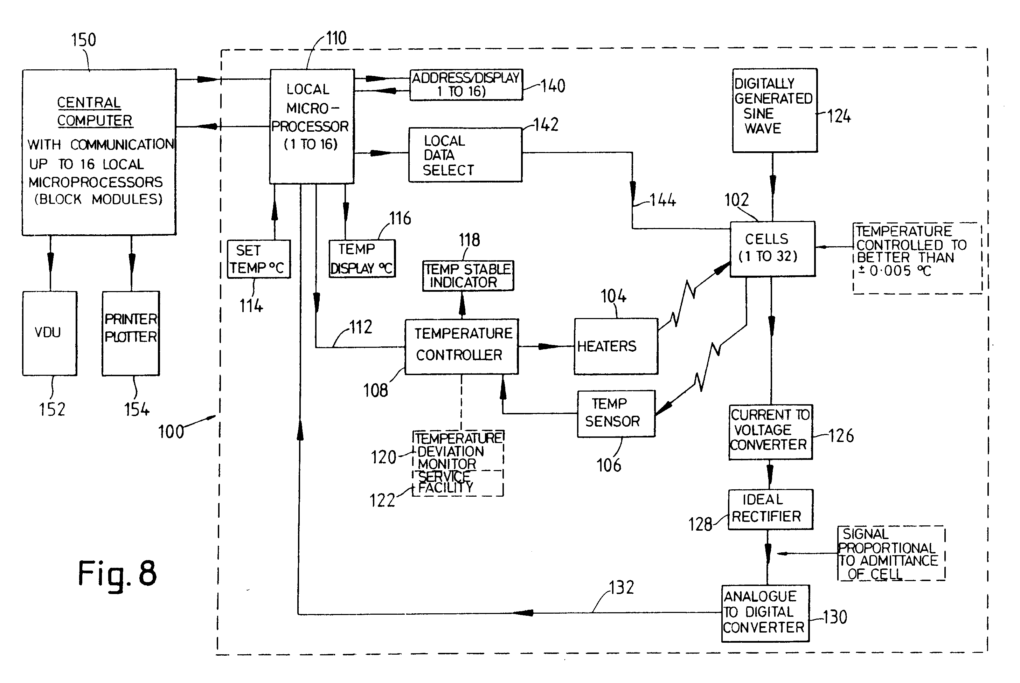

For each module or apparatus as thus far described, monitoring and control provisions are indicated in Figure 8 within the dashed block 100 showing a bank of cells 50 at 102, related heaters at 104 and temperature sensing at 106, and a temperature controller 108 operative relative to a demand signal shown coming from a local microprocessor controller 110 over line 112, there also being at least related temperature setting 114, and temperature display (116) and temperature stable indicator (118) provisions, perhaps also for temperature deviation and service/test facility shown dashed at 120 and 122.

Temperature control to within four-thousandths of one degree Celsius is readily achieved, and actual loading of test cells can be delayed until the temperature stable indicator 118 operates.

Bacteriological activity affects electrical impedance of a nutrient medium into which samples have been introduced in the test cells, specifically between the electrodes 54 of the plug 52. Apparatus being described monitors admittance and does so using a sine wave generator 124, conveniently of digital type, supplying the electrodes 54, a current-to-voltage converter 126, a rectifier 128 to give a signal proportional to admittance, and an analogue-to-digital converter 130 supplying a digital representation over line or lines 132.

Figure 8 further shows, in the module block 100, an address display means 140 and data select means 242 shown controlling interrogation of the test cells 102 over line 144. In practice, only relatively infrequent interrogation is required, say every few minutes, and it is particularly advantageous to have, or have the capability for, up to a considerable number say up to sixteen, of modules like 100, all under the control of a central computer 150 shown with associated visual display unit 152 and a printer/plotter 154 at a suitable console.

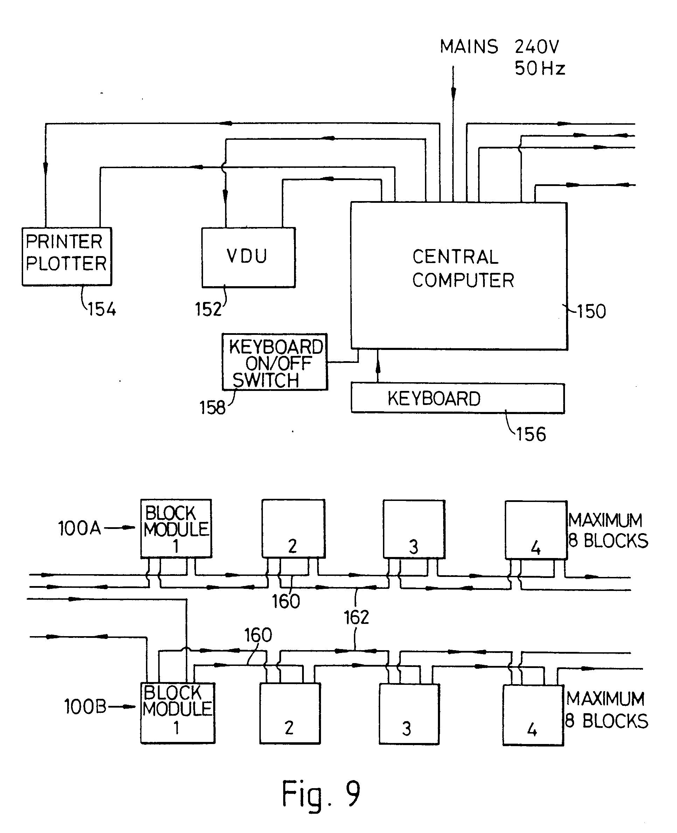

Such modular extension is shown in Figure 9, also showing a keyboard 156 for the control computer 150 and a keyboard switch 158, and for up to sixteen modules like 100, indicated organised in two banks 100A and 100B each of up to eight modules connected consecutively after the manner of a daisy-chain, but actually in a single daisy-chain. Connections comprise mains supply 160 (single-arrowed) and data communication 162 (double-arrowed).

It will be appreciated that different modules can be used for different purposes requiring different temperatures. Most often, however, in a practical installation, each module will not require change from one operating temperature to another, though that could obviously be done. If cooling was required, selective fan-operated means might be required unless long wait times are tolerable.

Regarding heating and temperature control, we find that no more than about 80 or 90 watts is required, and such is available from tubes made for fridges, and that proportional control is achievable using a single Wheatsone's bridge with the temperature sensing thermistor in one arm. As little as 0.05 degrees Celsius temperature gradient over an aluminium block in a casing hereof is readily achieved, at least at typical temperatures involved, often about 37 degrees Celsius.

Regarding response to change of impedance, a highly stable frequency signal of ten-kilohertz has proved satisfactory, say divided down from a one-megahertz crystal clock. At such frequency, for the sine wave applied to the test cells, capacitance is high (1-9 microfarads) compared to resistance (about 100 ohms), so that effects of the latter on admittance are small using simple detection circuitry - even an error of one ohm in one hundred is vastly reduced in its effect on admittance (to about 0.2-0.3%). Effectively, only two semiconductor integrated circuits (chips) are required for detection involving current-to-voltage and analogue-to-digital conversions.

Regarding actuation of the modules 110 for interrogation purposes, say on a polled basis, switching means responsive to addressing is preferably not of solid state type (which can be highly temperature sensitive), but rather of electromechanical type, conveniently reed relays available in dual in-line packaging (hence showing of two banks A and B in Figure 9).

It is found to be particularly advantageous for some temperature sensitive electrical/electronic components/devices to be mounted as aforesaid on a circuit board within the lower casing part 20, thus be subject equally to any and all temperature changes.

Discrimination of about 1 in 10,000 has been achieved.

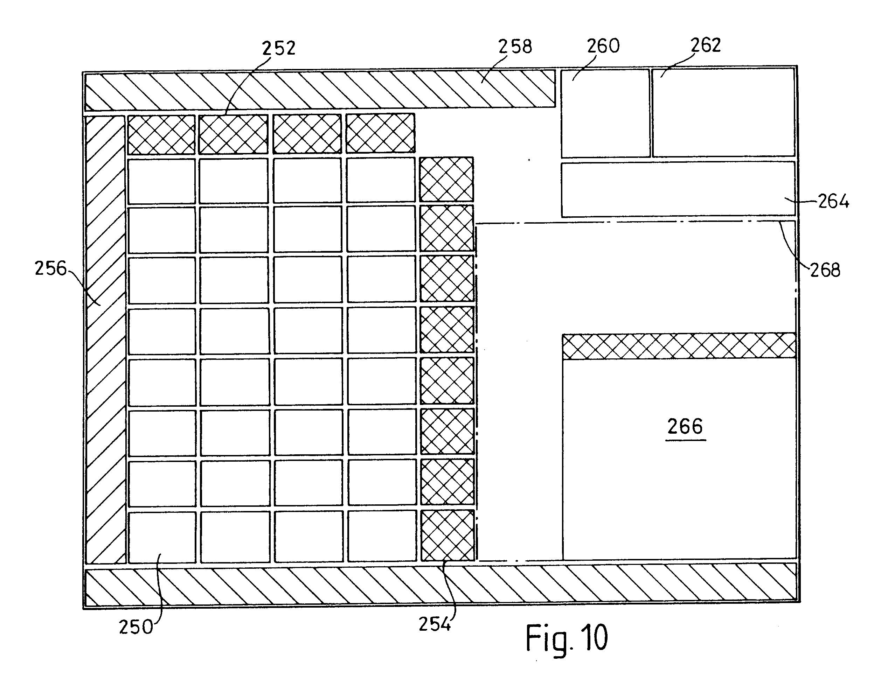

Turning to Figure 10, our particularly preferred screen presentation for ease of operator use is now described, i.e. at the visual display unit 152. It will, of course, be appreciated that the printer/plotter unit 154 will produce hard copy of progress and results, including graphical presentations as appropriate or desired.

However, the visual display unit is valuable, whether simply running in step with printer/plotter operations or, and particularly for operator use, selectively controlled from the keyboard 156.

The basic screen (200) format is correlated with each module, i.e. block 10 and associated test cells 50. To that end, a bank of four-by-eight display areas is defined at 250, one for each test cell, the bank 250 being flanked top and side (or bottom and side as an alternative) by areas 252 and 254, respectively, giving letters and numbers as references for each cell on a coordinate basis (or reversed, or two sets of numbers, or two sets of letters, if preferred). At the side of the screen next to the bank 250 is a strip 256 within which a cursor is defined to select any particular row, then a cell in that row (preferably to move onto that cell) by an arrow key or number key from the keyboard (or possibly another cursor in a strip below the bank 250). A top strip 258 is indicated above the bank 250 for information concerning what is being monitored. The right hand top corner is occupied by spaces 260,262 and 264 for diagnostic indication, time/date and temperature selected, respectively. The right-hand bottom corner has a block 266 containing a colour key relative to colours used for the constituent spaces of the block 250, i.e. for each cell. Finally, about the block 266, there is a region 268 in which further information can appear.

For a status interrogation at the VDU screen, different colours can be employed for indicating Test in Progress, Growth Detected, Fail, Suspect, Pass, Empty, Unknown Cell Present, Improperly Terminated. Moreover, it is feasible for the cell-spaces in block 250 also to carry information words, for example Completed or Running (then perhaps with elapsed time its indication).

## Claims

1. Bacterial growth testing apparatus comprising at least one heat conducting block (10) with through-holes (12) between opposite faces of the block that provide accommodations for plural test cells (50), respectively, and related controlled heating provision (at 14, 16), whereby test cells (50) in said through-holes (12) are surrounded by material of said block (10) maintained at a suitable temperature; Plural test cells (50), each of generally tubular form to extend from releasable electrical connectors (52, 60) at one of said faces for electric current to and from electrodes (54) internal of said cells (50), and each cell (50) protruding from the other of said faces; plural closures (70) for said cells (50) where protruding from said other face; and associated electronic monitoring and display means (150-154) for processing signals according to electrical current applied to said electrodes (54) as affected by bacterial activity in content of said cells (50), which monitoring and display means (150-154) is capable of servicing said one and other similar blocks (10) and associated test cells (50) on a modular extension basis for connections (160, 162) therebetween.

2. Apparatus according to claim 1, wherein said block (10) has a substantially centrally located blind hole (16) for temperature sensing means (106).

3. Apparatus according to claim 1 or claim 2, wherein said block (10) has counterbored through-holes for mounting purposes.

4. Apparatus according to claim 1,2 or 3, wherein heating and temperature control means (108) includes thermistor (106) bridge circuitry for proportional control.

5. Apparatus according to any preceding claim, wherein said releasable electrical connections each comprise co-operating parts of electrical connectors fixed (60) to the block (10) and detachably associated (52) with the cells(50), respectively.

6. Apparatus according to claim 5, wherein each (52) of said co-operating parts associated with the cells also carries said electrodes (54).

7. Apparatus according to claim 6, wherein said electrodes (54) are of stainless steel.

8. Apparatus according to any preceding claim, wherein each of said closures comprises an overfitting dished cap (70) having a slip-on fit to protruding ends of said cells (50) and an edge-engaging seal (80) to said ends, the cap (70) being displaceable by and to relieve gas pressure build up in an associated said cell (50).

9. Apparatus according to claim 8, wherein said cap is domed (80) inwardly to provide said seal.

10. Apparatus according to claim 8 or claim 9, wherein said cap has internal spacers (74) relative to said associated cell (50) for location and venting purposes.

11. Apparatus according to any preceding claim, further comprising a cabinet (20, 30) housing said block (10) with its one face at an angle rising from the user.

12. Apparatus according to any preceding claim, further comprising a cabinet (20, 30) housing said block (10) with at least some electronics (28) local to the block in a compartment (26) heat-shielded from said block (10).

13. Apparatus according to claim 11 or claim 12, wherein material of which the cabinet (20, 30) is made affords heat insulation for said block (10).

14. Apparatus according to 11, 12 or 13, wherein said cabinet is of upper (30) and lower (20) part construction, the lower part (20) carrying said block (10) and the upper part (30) fitting over said block (10) onto the lower part (20).

15. Apparatus according to claim 14, wherein said upper part (30) has holes (34) registering with the test cell accommodating through-holes (12) of said block (10) so that said test cells (50) also protrude therethrough.

16. Apparatus according to claim 15, wherein said upper part (30) is recessed (36A,B,C) and labelled or marked as to locations of said test cells (50).

17. Apparatus according to claim 15 or 16, wherein said upper part (30) and protruding said test cells (50) have a see-through cover (40) thereover.

18. Apparatus according to any preceding claim, wherein electrical impedance sensing for indicating bacterial activity involves applying a highly stable frequency electrical signal to said test cells (50).

19. Apparatus according to claim 18, wherein said frequency is at 10 KiloHertz.

20. Apparatus according to any preceding claim, wherein plural modules (100) have electromechanical switching means responsive to addressing thereof.

21. Apparatus according to any preceding claim, wherein display means (152) is controlled and operative to show a bank of areas (250) corresponding to said test cells (50) of a said block (10), and for colouring those areas (250) according to last interrogated status of corresponding said test cells (50).

22. Apparatus according to claim 21, wherein said display means (152) has a dedicated area (266) to display a colour key for said colouring according to test cell status.

## Patentansprüche

1. Apparat zum Testen bakteriellen Wachstums, aufweisend:
- mindestens einen Wärmeleitblock (10) mit durchgehenden Bohrungen (12) zwischen den gegenüberliegenden Flächen des Blocks zur Unterbringung von mehreren Reagenzbehältern (50) und entsprechenden gesteuerten Heizvorrichtungen (bei 14, 16), wobei die Reagenzbehälter (50) in den genannten Bohrungen (12) vom Material des genannten Blocks (10) umgeben sind und auf geeigneter Temperatur gehalten werden;
- mehrere, im allgemeinen röhrenförmige Reagenzbehälter (50), die von lösbaren elektrischen Anschlüssen (52, 60) auf der einen der genannten Flächen zur Zu- und Ableitung elektrischen Stroms in die und aus den Elektroden (54) im Inneren der genannten Behälter (50) ausgehen und wobei jeder Behälter (50) am anderen Ende der genannten Flächen übersteht;
- Mehrfachverschlüsse (70) für die genannten Behälter auf der Seite, auf der sie gegen die genannte andere Fläche überstehen und
- zugehörige elektronische Beobachtungs- und Darstellungsmittel (150-154) zur Verarbeitung der Signale entsprechend dem zwischen beiden Elektroden (54) fließenden elektrischen Strom je nach bakterieller Aktivität in den genannten Behältern (50), wobei die Beobachtungs- und Darstellungsmittel (150, 154) in der Lage sind, den genannten und weitere ähnliche Blöcke (10) mit zugehörigen Reagenzbehältern (50) durch modulare Erweiterung über die dazwischenliegenden Anschlüsse (160, 162) zu bedienen.

2. Apparat gemäß Anspruch 1, wobei der genannte Block (10) ein im wesentlichen mittig angeordnetes Grundloch (16) für Temperaturfühler (106) besitzt.

3. Apparat gemäß Anspruch 1 oder Anspruch 2, wobei der genannte Block (10) angesenkte Durchbohrungen für Montagezwecke aufweist.

4. Apparat gemäß Anspruch 1, 2 oder 3, wobei zu Heizung und Temperaturüberwachungsmittel (108) eine Thermistor (106) - Brückenschaltung zur Proportionalsteuerung gehört.

5. Apparat gemäß einem der vorangegangenen Ansprüche, wobei die genannten lösbaren elektrischen Anschlüsse jeweils fest am Block (10) montierte zusammenwirkende Teile elektrischer Steckverbindungen (60) umfassen, die jeweils lösbar mit den Behältern (52) verbunden sind (52).

6. Apparat gemäß Anspruch 5, wobei jedes (52) der genannten zusammenwirkenden und zu den Behältern gehörenden Teile außerdem die genannten Elektroden (54) trägt.

7. Apparat gemäß Anspruch 6, wobei die genannten Elektroden (54) aus Edelstahl sind.

8. Apparat gemäß einem der vorangegangenen Ansprüche, wobei jeder der genannten Verschlüsse eine gewölbte auf die hervorstehenden Enden der genannten Behälter (50) aufsteckbare Kappe (70) mit Randdichtung (80) an den genannten Enden umfaßt und die Kappe (70) zur Druckentlastung im Inneren des zugehörigen genannten Behälters (50) durch eben diesen Druck verschoben werden kann.

9. Apparat gemäß Anspruch 8, wobei die genannte Kappe zur Gewährleistung der genannten Dichtung nach innen gewölbt ist (80).

10. Apparat gemäß Anspruch 8 oder Anspruch 9, wobei die genannte Kappe innen mit Abstandhaltern (74) zu dem zugehörigen Behälter (50) zur Anbringung und Belüftung versehen ist.

11. Apparat gemäß einem der vorangegangenen Ansprüche, der außerdem einen Kasten (20, 30) umfaßt, in dem der genannte Block (10) untergebracht ist und dessen eine Fläche in einem Winkel von vorn her ansteigt.

12. Apparat gemäß einem der vorangegangenen Ansprüche, der außerdem einen Kasten (20, 30) umfaßt, in dem der genannte Block (10) untergebracht ist, mit mindestens etwas Elektronik (28) für den Block in einer gegenüber dem genannten Block (10) wärmeisolierten Kammer (26)

13. Apparat gemäß Anspruch 11 oder Anspruch 12, wobei das Material des Kastens (20, 30) für eine Wärmeisolierung des genannten Blocks (10) sorgt.

14. Ein Aparat gemäß Anspruch 11, 12oder 13, wobei der genannte Kasten aus einem Oberteil (30) und einem Unterteil (20) besteht, wobei das Unterteil (20) den genannten Block (10) trägt, und das Oberteil (30) über den genannten Block (10) auf das Unterteil (20) gesteckt ist.

15. Apparat gemäß Anspruch 14, wobei das genannte Oberteil (30) mit den Durchbohrungen (12) im Block (10) zur Unterbringung der Reagenzbehälter übereinstimmende Löcher (34) aufweist, so daß die genannten Behälter (50) aus diesen ebenfalls hervorstehen.

16. Apparat gemäß Anspruch 15, wobei das genannte Oberteil (30) Vertiefungen (36A, B, C) aufweist sowie mit Etiketten oder Markierungen in bezug auf die Anordnung der genannten Reagenzbehälter (50) versehen ist.

17. Apparat gemäß Anspruch 15 oder 16, wobei sich über dem genannten Oberteil (30) und den hervorstehenden genannten Reagenzbehältern (50) ein durchsichtiger Deckel (40) befindet.

18. Apparat gemäß einem der vorangegangenen Ansprüche, wobei zur Auswertung der elektrischen Impedanz zur Sichtbarmachung der bakteriellen Aktivität ein elektrisches Signal mit einer hochstabilen Frequenz an die genannten Reagenzbehälter (50) angelegt wird.

19. Apparat gemäß Anspruch 18, wobei die genannte Frequenz 10 kHz beträgt.

20. Apparat gemäß einem der vorangegangenen Ansprüche, wobei Mehrfachmodule (100) einzeln ansteuerbare elektromechanische Schalter besitzen.

21. Apparat gemäß einem der vorangegangenen Ansprüche, wobei Anzeigen (152) gesteuert werden, um einen den genannten Reagenzbehältern (50) eines genannten Blocks (10) entsprechenden Flächenbereich (250) darzustellen sowie zur farblichen Gestaltung dieser Flächenbereiche (250) gemäß der letzten Statusabfrage der entsprechenden genannten Reagenzbehälter (50).

22. Apparat gemäß Anspruch 21, wobei die genannten Anzeigen (152) über eine zugewiesene Fläche (266) zur Darstellung eines Farbschlüssels für die genannte Farbgebung entsprechend dem Status der Reagenzbehälter verfügen.

## Revendications

1. Dispositif de test de croissance bactériologique comportant au moins un bloc (10) conducteur de la chaleur et percé entre deux de ses faces opposées de trous (12) pour loger des cellules de test multiples (50), respectivement, et des points associés de chauffage contrôlé (14,16), ces cellules de test (50) logées dans les trous (12) étant ainsi entourées par la matière du bloc (10) qui est maintenue à une température appropriée, ces cellules de test multiples (50) ayant chacune une forme sensiblement tubulaire, et s'étendant à partir de connexions électriques amovibles (52,60) situées à l'endroit de l'une des faces du bloc, pour le passage d'un courant électrique dans des électrodes (54) prévues à l'intérieur desdites cellules (50), et ces cellules (50) débordant en saillie sur l'autre face du bloc; des moyens d'obturation multiples (70) étant prévus pour lesdites cellules (50) en saillie sur l'autre face du bloc, ainsi que des moyens électroniques associés (150-154) de surveillance et de représentation, pour traiter les signaux dus au courant électrique appliqué auxdites électrodes (54) et affecté par l'activité bactériologique du contenu desdites cellules (50), ces moyens de surveillance et de représentation (150-154) permettant de desservir le bloc en question et d'autres blocs semblables (10) avec leurs cellules de test (50), sur le principe d'un système d'extension modulaire des lignes de connexion (160, 162) entre ces blocs.

2. Dispositif selon la revendication 1, dans lequel ledit bloc (10) comporte un trou borgne sensiblement central (16) pour un capteur de température (106).

3. Dispositif selon la revendication 1 ou 2, dans lequel ledit bloc (10) comporte des trous percés de part en part du bloc et alésés pour le montage de celui-ci.

4. Dispositif selon la revendication 1, 2 ou 3, dans lequel les moyens de contrôle du chauffage et de la température (108) comportent un thermistor (106) associé à un circuit à pont pour réaliser un système de commande proportionnelle.

5. Dispositif selon l'une quelconque des revendications précédentes, dans lequel lesdites connexions électriques amovibles comportent chacune des organes de contact électrique (60) fixés au bloc (10) et associés par une liaison amovible (52) à chacune des cellules (50), respectivement.

6. Dispositif selon la revendication 5, dans lequel chacune desdites pièces de liaison (52) associées aux cellules porte également lesdites électrodes (54).

7. Dispositif selon la revendication 6, dans lequel lesdites électrodes (54) sont en acier inoxydable.

8. Dispositif selon l'une quelconque des revendications précédentes, dans lequel chacun desdits moyens d'obturation comporte un capuchon (70) adapté à coiffer l'extrémité en sailllie de la cellule à obturer (50) en glissant sur cette extrémité, et un joint (80) prévu pour s'appuyer sur le bord de celle-ci, ce capuchon (70) pouvant se déplacer sous l'effet de la pression gazeuse produite dans la cellule de test associée (50) afin de réduire cette pression.

9. Dispositif selon la revendication 8, dans lequel ledit capuchon présente vers l'intérieur une forme bombée (80) pour réaliser ledit joint.

10. Dispositif selon l'une des revendications 8 ou 9, dans lequel ledit capuchon présente intérieurement des bossages d'espacement (74) par rapport à la cellule associée (50) pour assurer son positionnement et permettre le dégazage.

11. Dispositif selon l'une quelconque des revendications précédentes, comportant en outre un coffret (20,30) pour loger le bloc (10), ce coffret ayant une face inclinée en regard de l'utilisateur.

12. Dispositif selon l'une quelconque des revendications précédentes, comportant en outre un coffret (20,30) pour loger le bloc (10) avec au moins certains organes électroniques (28) associés au bloc et disposés dans un compartiment (26) qui est isolé thermiquement par rapport au bloc (10).

13. Dispositif selon la revendication 11 ou 12, dans lequel la matière constitutive du coffret (20,30) assure l'isolation thermique du bloc (10).

14. Dispositif selon l'une des revendications 11, 12 ou 13, dans lequel le coffret comporte une partie supérieure (30) et une partie inférieure (20), cette partie inférieure (20) contenant le bloc (10), et la partie supérieure (30) se montant par-dessus le bloc (10) en coiffant la partie inférieure (20).

15. Dispositif selon la revendication 14, dans lequel ladite partie supérieure (30) du coffret présente des trous (34) qui coïncident avec les trous (12) ménagés de part en part du bloc (10) pour loger les cellules de test (50), les parties en saillie de ces cellules venant ainsi se loger dans les trous (34) de la partie supérieure (30) du coffret.

16. Dispositif selon la revendication 15, dans lequel ladite partie supérieure (30) du coffret présente des logements en creux (36A,B,C) pour des étiquettes ou des marques indiquant l'emplacement des cellules de test (50).

17. Dispositif selon la revendication 15 ou 16, dans lequel un couvercle transparent (40) est prévu pour recouvrir ladite partie supérieure (30) du coffret et les parties en saillie des cellules de test (50).

18. Dispositif selon l'une quelconque des revendications précédentes, dans lequel sont prévus des moyens pour détecter l'impédance électrique traduisant une activité bactériologique, en appliquant aux cellules de test (50) un signal électrique sous une fréquence de haute stabilité.

19. Dispositif selon la revendication 18, dans lequel la fréquence est de 10 kiloHertz.

20. Dispositif selon l'une quelconque des revendications précédentes, dans lequel sont prévus des modules multiples (100) associés à un système de commutation électro-mécanique leur permettant de répondre lorsqu'on les interroge.

21. Dispositif selon l'une quelconque des revendications précédentes, dans lequel un système de représentation (152) actionné par un opérateur, est prévu pour faire apparaître sur un écran des plages (250) correspondant à une rangée de cellules de test (50) du bloc (10), et pour colorer ces plages (250) d'après l'état des cellules d'essai correspondantes (50) au moment de la dernière interrogation.

22. Dispositif selon la revendication 21, dans lequel l'écran du système de représentation (152) comporte une zone réservée (266) pour faire apparaître un code de couleurs correspondant aux états des cellules de test.
